# EUROPEAN PATENT SPECIFICATION

(11) **EP 0 567 444 B1**
(45) Date of publication and mention of the grant of the patent: **20.03.1996**
(21) Application number: 93870056.4
(22) Date of filing: 26.03.1993
(51) Int. Cl.: C07C 5/27, C07C 5/41

(54) **Aromatization and isomerization of hydrocarbons**
Verfahren zur Aromatisierung und Isomerisierung von Kohlenwasserstoffen
Procédé pour l'aromatisation et l'isomérisation d'hydrocarbures

(30) Priority: 26.03.1992 US 857860
(43) Date of publication of application: 27.10.1993
(73) Proprietor: FINA TECHNOLOGY, INC., Dallas, Texas 75206 (US)
(72) Inventor: Shamshoum, Edwar, Houston, Texas 77062 (US); Ghosh, Ashim, Houston, Texas 77062 (US); Schuler, Thomas, Galena Park, Texas 7745 (US)
(74) Representative: Leyder, Francis

(56) References cited:
- FR-A- 2 183 520
- US-A- 4 962 250

## Description

### TECHNICAL FIELD OF THE INVENTION

This invention relates to a process employing a platinum metal loaded X zeolite conversion catalyst for the aromatization of paraffinic feedstocks. Prior to platinum metal loading, the X zeolite is fully ion-exchanged with calcium ions. In addition to the aromatic products, desirable isoparaffins are also produced.

### BACKGROUND OF THE INVENTION

Various prior art processes involve the conversion of paraffinic hydrocarbons to aromatics. For example, U.S. Patent No. 4,347,395 to Chu et al. discloses a process for producing aromatic compounds by contact of a gaseous hydrocarbon feedstock containing predominantly paraffinic hydrocarbons of two (2) to six (6) carbon atoms with oxygen or air in the presence a catalyst system to convert at least a portion of the feed hydrocarbons to liquid aromatic hydrocarbons. The catalyst system comprises a crystalline zeolite catalyst in combination with a metal or metal oxide oxidative dehydrogenation component. Suitable dehydrogenation components include ferric oxide, potassium oxide, and chromium oxide and a mixture of iron ferrite and an oxide of a metal selected from the group consisting of cerium, zinc, manganese, lead, and mixtures thereof. Other oxidative dehydrogenation components include a combination of the oxides of chromium, molybdenum and phosphorous or the oxides of niobium, vanadium and molybdenum. The zeolite and oxidative dehydrogenation component can be present in separate zones, in a single zone, or preferably a composite mixture of the two is prepared by ion-exchange or at least partial impregnation of the dehydrogenation component into the zeolite material.

U.S. Patent No. 4,403,044 to Post, et al. discloses a large number of conversion techniques including aromatization procedures employing feedstreams selected from the class of carbon monoxide and hydrogen mixtures, acyclic organic compounds, aliphatic and/or cycloaliphatic hydrocarbons and mixtures thereof. A wide variety of silicalite based catalyst systems are disclosed for use in the conversion techniques of Post, et al. The catalyst systems, based upon silicalite as the carrier, include metal or metal combinations of nickel, copper, zinc, cadmium, platinum, palladium, nickel-tungsten, cobalt-molybdenum, nickel-molybdenum, zinc-palladium, zinc-copper, and zinc-rhenium. Other metal combinations include iron-chromium oxide, and zinc oxide-chromium oxide. Deposition of the metal combinations on the silicalite may be by impregnation.

U.S. Patent No. 3,760,024 to Cattanach discloses the conversion of a feed consisting essentially of C₂-C₄ paraffins and/or olefins to aromatics in the presence of a crystalline aluminosilicate catalyst of the ZSM-5 type.

In a typical refinery operation, normal hexane and pentane paraffins are isolated and fed into a Total Isomerization Process (TIP) unit to produce higher octane isoparaffins. The heptanes, octanes and heavier paraffins are usually blended in the JP-4 jet fuel. It would be desirable, however, to upgrade such heptanes, octanes, etc., via an aromatization and isomerization process to produce higher octane aromatics, such as toluene, xylenes, and ethylbenzene, as well as isoparaffins.

### SUMMARY OF THE INVENTION

In accordance with the present invention, there is provided a process for the conversion of normal paraffins to more valuable aromatic and isoparaffinic compounds. The present invention may be utilized to obtain high liquid yields of aromatic products having significantly higher octane (RON) values than the paraffinic feed, while at the same time also producing substantial amounts of octane enhancing isoparaffins. The process comprises contacting a hydrocarbon feedstock containing normal paraffins with a hydrocarbon conversion catalyst under conversion conditions whereby aromatic and isoparaffinic products are formed. The hydrocarbon conversion catalyst is a platinum metal-loaded X zeolite having calcium ions incorporated in the cage structure thereof.

The platinum/calcium X (Pt/CaX) zeolite catalyst useful in the present process is preferably prepared by first fully ion-exchanging an "as synthesized" X zeolite with calcium ions, and then ion-exchanging this calcium containing form of the zeolite with platinum ions. The platinum and calcium ion-exchanged zeolite is extruded with a binder, such as alumina or silica, and is then calcined. Alternatively, platinum can be incorporated into the catalyst by wet impregnation of the calcium X zeolite after it has been extruded with a binder.

### DETAILED DESCRIPTION OF THE INVENTION

The present invention relates to a process for the aromatization and isomerization of normal paraffins to aromatic and isoparaffin products. The process employs a hydrocarbon conversion catalyst comprising a platinum/calcium X (Pt/CaX) zeolite containing from about 0.2 to 2.0 weight percent platinum and from about 1 to 10 weight percent calcium, with 0.4 to 1.0 weight percent platinum and 5.0 to 7.0 weight percent calcium being preferred.

In one embodiment, a hydrocarbon feedstock containing normal paraffins is supplied to a reaction zone where it is brought into contact with a Pt/CaX zeolite conversion catalyst under conversion reaction conditions effective to cause aromatization of the normal paraffins, thereby producing liquid aromatic products having a RON value greater than that of the paraffin feed. Hydrogen is also supplied to the reaction zone, preferably as a co-feed with the paraffin-containing hydrocarbon feed. Preferred hydrogen/hydrocarbon feed molar ratios are from about 1 to 10, with about 3 to 5 more preferred. Conversion reaction conditions include a temperature between about 300°C and 600°C, with about 425°C to 525°C being preferred, and a pressure between about 689500 Pa and 3447500 Pa (100 and 500 psig), with about 1379000 Pa (200 psig) being preferred. Additionally, liquid hourly space velocities (LHSV) from about 0.5 to 5.0 are utilized for the hydrocarbon feed, with an LHSV of approximately 1 to 3 being preferred.

The present invention has utility for the conversion of hydrocarbon feedstocks containing normal C₅ to C₁₂ paraffins.

Aromatic product yields of up to about 40 wt. % can be obtained, and the unconverted feed can be repeatedly recycled if desired. The specific aromatic products formed, of course, will vary depending on the hydrocarbon feed. For example, a feed consisting of mostly C₇ and C₈ paraffins will yield a liquid aromatic product containing toluene, ethylbenzene and xylenes, although minor amounts of benzene may also be produced. In addition to converting the normal paraffins to aromatic compounds, significant amounts of isoparaffins will also be produced. Even when operating within the preferred conversion condition ranges most suitable for aromatization, isoparaffin yields in excess of 25 wt.% can be obtained.

A Pt/CaX zeolite catalyst suitable for use in the present invention can be prepared by modification of a "parent" crystalline X zeolite having a silica to alumina (SiO₂/Al₂O₃) molar ratio between about 1 and 3. Basic procedures for the preparation of such a "parent" X zeolite are well known in the art and need not be detailed herein. In accordance with a preferred modification method, the parent X zeolite is first subjected to repeated calcium ion-exchanges to obtain the maximum loading of calcium ions in the cage structure of the zeolite. An aqueous solution of an inorganic calcium salt, preferably calcium nitrate, is employed as the ion-exchange medium.

This calcium-containing form of the X zeolite powder is then subjected to platinum ion exchange. Such platinum ion-exchange is preferably accomplished by utilization of an aqueous platinum salt solution, such as tetraamineplatinum chloride. After the calcium and platinum ions have been incorporated into the X zeolite, it is then mixed with a binder, such as silica, alumina sol, gamma-alumina or other refractory oxides. This mixture is then pelletized by a suitable technique, such as extrusion, and the resulting pellets calcined at a maximum temperature of 530°C. The resulting Pt/CaX zeolite preferably contains from about 0.2 to 2.0 weight percent platinum and 1 to 10 weight percent calcium prior to extrusion with the binder, with 0.4 to 1.0 weight percent platinum and 5.0 to 7.0 weight percent calcium being more preferred.

An alternative method for incorporating platinum into the catalyst is via wet impregnation of the calcium-containing X zeolite after extrusion with the binder. Tetraamineplatinum chloride preferably is employed as the wet impregnation medium.

Practice of the present process within the preferred reaction conditions specified above will generally yield an aromatic product in an amount of approximately 10 to 40 wt.%. Specific liquid yields and RON values obtained in accordance with the present invention will depend primarily upon the reaction temperature, the selection of which may be governed by economic conditions. For example, by operating at a reaction temperature between approximately 500°C to 525°C, one can obtain a liquid yield (C₅₊) in excess of 70 weight percent having a RON greater than 70. If a higher liquid yield is desired, a lower reaction temperature should be employed. Of course, employing a lower temperature will also result in a lower RON. For example, operating at a temperature between about 435°C to 470°C, one can obtain a liquid yield of approximately 90 weight percent having a RON greater than 30.

The following experimental work will serve to more fully describe the present invention. It is understood that these examples are not intended to limit the true scope of the invention, but rather are presented for illustrative purposes.

In experimental work carried out in accordance with the present invention, three Pt/CaX zeolite catalysts and one platinum/potassium X (Pt/KX) zeolite catalyst were employed for the conversion of a mixed feed containing normal heptane (nC₇) and normal octane (nC₈) to aromatic and isoparaffinic products (iC₇ and iC₈). Two of the Pt/CaX zeolite catalysts differed only with respect to the binder employed, with one having 20 wt. % alumina (Pt/CaX-I), and the other, 20 wt. % silica (Pt/CaX-II). The third Pt/CaX zeolite catalyst (Pt/CaX-III) also contained 20 wt. % silica as the binder, but in this case, platinum was incorporated into the catalyst by wet impregnation of the calcium X zeolite after it was extruded with the binder.

### EXAMPLE 1

### (Preparation of Calcium X Zeolite)

A parent, or "as synthesized" X zeolite was fully ion-exchanged with calcium ions in an aqueous solution as follows: About 272g of the zeolite X powder was added in 3ℓ deionized water in a flux and was then heated (to 100°C) and stirred. About 420g of calcium nitrate was added to the zeolite suspension. Ion-exchange was continued for at least 4 hours at 100°C, after which the zeolite powder was filtered, washed and dried. A total of three successive calcium ion-exchanges were given to obtain a maximum Ca ion loading in the zeolite.

### EXAMPLE 2

### (Preparation of Pt/CaX-I)

The CaX zeolite powder prepared in EXAMPLE 1 was ion-exchanged with Pt ions using approximately 80g CaX powder and 0.70g tetraamineplatinum chloride. After filtration, washing and drying, the Pt/CaX zeolite catalyst was extruded with 20 wt. % alumina as a binder and calcined at a maximum temperature of 530°C for two hours.

### EXAMPLE 3

### (Preparation of Pt/CaX-II)

A Pt/CaX zeolite was prepared in a manner like that set forth in EXAMPLE 2 above, except that the Pt/CaX zeolite was extruded with 20 wt. % silica (instead of alumina) as the binder.

### EXAMPLE 4

### (Preparation of Pt/CaX-III)

A calcium ion-exchanged X zeolite prepared by a method like that set forth in EXAMPLE 1 above, was extruded with 20 wt. % silica as a binder. Platinum was then incorporated into the zeolite by wet impregnation as follows:
The water adsorption capacity of the extruded sample was first determined (0.88 ml/g catalyst). Then 0.142 g tetraamine-platinum chloride was dissolved in approximately 18 ml water and added to 20g of dried extrudate (SiO₂ bound) sample to fully wet. The sample was then dried at 110°C and re-calcined at 400°C.

### EXAMPLE 5

### (Preparation of Pt/KX)

A Pt/KX zeolite catalyst was prepared by a method like that set forth in EXAMPLE 2 above, except that the "parent" X zeolite was fully ion-exchanged with potassium ions (instead of calcium ions). Three successive ion-exchanges in potassium nitrate were employed to give a maximum loading of K ions in the zeolite. After platinum ion exchange, filtration and drying, the KX zeolite was also extruded with 20 wt. % alumina as a binder and calcined.

### EXAMPLE 6

### (Conversion of Normal Paraffins)

The catalysts prepared in EXAMPLES 2 through 5, above, were employed for the conversion of a mixed hydrocarbon feed containing approximately 60 wt. % normal heptane (nC₇) and 40 wt. % normal octane (nC₈) to aromatic and isoparaffinic (iC₇ and iC₈) products. The results obtained are listed below in TABLE I.

The following conversion procedure was utilized: Approximately 35 ml of the catalyst was loaded into a micro-reactor and activated by heating under hydrogen flow (0.4 l/min.), at a rate of 75°C per hour for the first two hours, followed by 50°C per hour until 350°C was reached. The system was left at this temperature overnight to complete activation. After the temperature indicated below was reached, the feed containing 60 wt. % nC₇ and 40 wt. % nC₈ was introduced at a rate to give a LHSV of approximately 1.8 hr⁻¹. A reaction pressure of 1379000 Pa (200 psig) was utilized. Hydrogen was used as a co-feed and was adjusted to give an hydrogen/hydrocarbon feed molar ratio of approximately 4.6. The reactor temperature was increased by 10°C increments until the maximum temperature indicated below was reached.

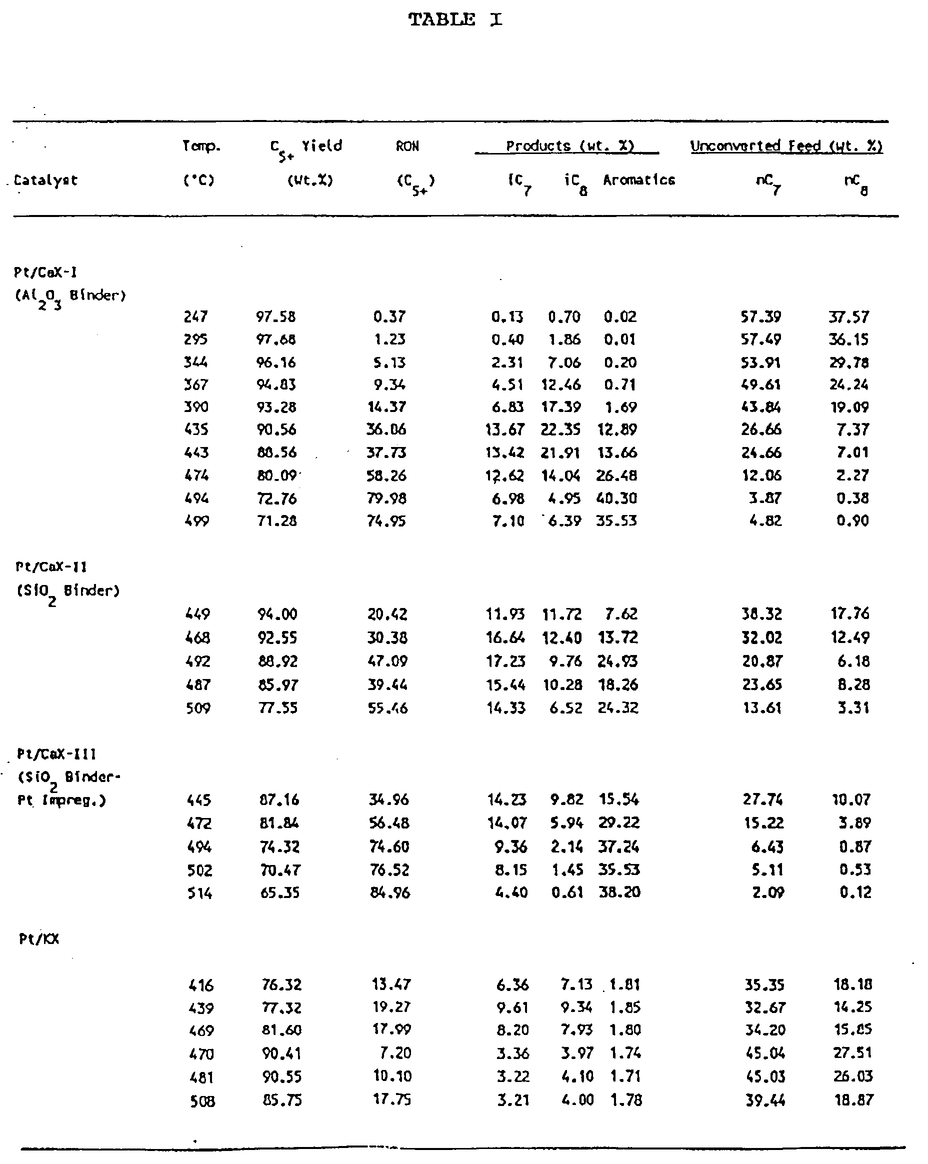

Having described specific embodiments of the present invention, it will be understood that modification thereof may be suggested to those skilled in the art, and it is intended to cover all such modifications as falls within the scope of the appended Claims.

## Claims

1. A process for the aromatization of normal paraffins comprising passing a hydrocarbon feedstream containing the normal paraffins through a reaction zone containing a platinum/calcium x zeolite catalyst under conversion conditions which comprise:
(a) Temperatures in the range of 300°C to 600°C;
(b) Pressures in the range of 689500 Pa to 3447500 Pa (100 psig to 500 psig); and
(c) Liquid hourly space velocities of the hydrocarbon feedstream between 0.5 and 5.0
and then recovering a liquid C₅₊ product containing aromatic hydrocarbons.

2. The process as recited in Claim 1 wherein the liquid C₅₊ product has a research octane number (RON) greater than that of the hydrocarbon feedstream.

3. The process as recited in Claim 1 wherein the normal paraffins have between five (5) and twelve (12) carbon atoms per molecule.

4. The process a recited in Claim 1 wherein hydrogen is supplied to the reaction zone as a co-feed with the hydrocarbon feedstock at a hydrogen/hydrocarbon feedstock molar ratio between 1 and 10.

5. The process as recited in Claim 1 wherein the liquid C₅₊ product represents at least 70 weight percent, based upon the normal paraffin feed.

6. The process as recited in Claim 1 wherein the liquid C₅₊ product has a research octane number (RON) of at least 50.

7. The process as recited in Claim 5 wherein the liquid C₅₊ product represents at least 90 weight percent, based upon the normal paraffin feed.

8. The process as recited in Claim 1 wherein the liquid C₅₊ product has a research octane number (RON) of at least 30.

9. The process as recited in Claim 1 wherein the liquid C₅₊ product also contains isoparaffins.

10. The process as recited in Claim 2 wherein the platinum/calcium X zeolite catalyst contains between 0.2 and 2.0 weight percent platinum and has between 5.0 and 7.0 weight percent calcium incorporated into the cage structure thereof.

## Patentansprüche

1. Verfahren zur Aromatisierung von normalen Paraffinen, umfassend Hindurchführen eines die normalen Paraffine enthaltenden Kohlenwasserstoffausgangsmaterialstroms durch eine einen Platin / Calcium X Zeolith-Katalysator enthaltende Reaktionszone unter Umwandlungsbedingungen, die umfassen:
(a) Temperaturen im Bereich von 300 °C bis 600 °C;
(b) Drücke im Bereich von 689500 Pa bis 3447500 Pa (100 psi Überdruck bis 500 psi Überdruck); und
(c) Flüssigkeitsstundenraumgeschwindigkeiten des Kohlenwasserstoffausgangsmaterialstroms zwischen 0,5 und 5,0
und dann Gewinnen eines flüssigen C₅₊ Produkts, das aromatische Kohlenwasserstoffe enthält.

2. Verfahren nach Anspruch 1, wobei das flüssige C₅₊ Produkt eine größere Research Oktanzahl (ROZ) aufweist als der Kohlenwasserstoffausgangsmaterialstrom.

3. Verfahren nach Anspruch 1, wobei die normalen Paraffine zwischen fünf (5) und zwölf (12) Kohlenstoffatome pro Molekül aufweisen.

4. Verfahren nach Anspruch 1, wobei der Wasserstoff als Co-Ausgangsmaterial mit dem Kohlenwasserstoffausgangsmaterial bei einem molaren verhältnis von Wasserstoff / Kohlenwasserstoffausgangsmaterial zwischen 1 und 10 in die Reaktionszone gebracht wird.

5. Verfahren nach Anspruch 1, wobei das flüssige C₅₊ Produkt bezogen auf das Ausgangsmaterial des normalen Paraffins mindestens 70 Gew.-% ausmacht.

6. Verfahren nach Anspruch 1, worin das flüssige C₅₊ Produkt eine Research Oktanzahl (ROZ) von mindestens 50 aufweist.

7. Verfahren nach Anspruch 5, wobei das flüssige C₅₊ Produkt bezogen auf das Ausgangsmaterial des normalen Paraffins mindestens 90 Gew.-% ausmacht.

8. Verfahren nach Anspruch 1, wobei das flüssige C₅₊ Produkt eine Research Oktanzahl (ROZ) von mindestens 30 aufweist.

9. Verfahren nach Anspruch 1, wobei das flüssige C₅₊ Produkt auch Isoparaffine enthält.

10. Verfahren nach Anspruch 2, wobei der Platin / Calcium X Zeolith-Katalysator zwischen 0,2 und 2 Gew.-% Platin enthält und zwischen 5,0 und 7,0 Gew.-% in dessen Käfigstruktur eingelagertes Calcium aufweist.

## Revendications

1. Procédé d'aromatisation d'hydrocarbures paraffiniques normaux comprenant
le passage d'une charge hydrocarbonée contenant les hydrocarbures paraffiniques normaux dans une zone de réaction qui contient un catalyseur zéolite x de platine/calcium sous des conditions de conversion qui comprennent:
(a) des températures dans la gamme de 300°C à 600°C;
(b) des pressions dans la gamme de 689500 Pa à 3447500 Pa (100 psig à 500 psig); et
(c) des vitesses spatiales liquides horaires de la charge hydrocarbonée comprises entre 0,5 et 5,0
et ensuite la récupération d'un produit liquide C₅₊ contenant les hydrocarbures aromatiques.

2. Procédé tel que récité dans la revendication 1 dans lequel le produit liquide C₅₊ a un indice d'octane recherche (RON) plus grand que celui de la charge hydrocarbonée.

3. Procédé tel que récité dans la revendication 1 dans lequel les hydrocarbures paraffiniques normaux ont entre cinq (5) et douze (12) atomes de carbone par molécule.

4. Procédé tel que récité dans la revendication 1 dans lequel l'hydrogène est alimenté dans la zone de réaction comme co-charge avec la charge hydrocarbonée avec un rapport molaire hydrogène/charge hydrocarbonée compris entre 1 et 10.

5. Procédé tel que récité dans la revendication 1 dans lequel le produit liquide C₅₊ représente au moins 70 pour-cent en poids, basé sur la charge d'hydrocarbures paraffiniques normaux.

6. Procédé tel que récité dans la revendication 1 dans lequel le produit liquide C₅₊ a un indice d'octane recherche (RON) d'au moins 50.

7. Procédé tel que récité dans la revendication 5 dans lequel le produit liquide C₅₊ représente au moins 90 pour-cent en poids, basé sur la charge d'hydrocarbures paraffiniques normaux.

8. Procédé tel que récité dans la revendication 1 dans lequel le produit liquide C₅₊ a un indice d'octane recherche (RON) d'au moins 30.

9. Procédé tel que récité dans la revendication 1 dans lequel le produit liquide C₅₊ contient aussi des isoparaffines.

10. Procédé tel que récité dans la revendication 2 dans lequel le catalyseur zéolite X de platine/calcium contient entre 0,2 et 2,0 pour-cent en poids de platine et a entre 5,0 et 7,0 pour-cent en poids de calcium incorporé dans sa structure de cage.
